# EUROPEAN PATENT APPLICATION

(11) **EP 4 650 746 A1**
(43) Date of publication of application: **19.11.2025**
(21) Application number: 24176716.9
(22) Date of filing: 17.05.2024
(51) Int. Cl.: G01N 1/20, G01N 1/22, G01N 33/00

(54) **EXTRACTION PROBE FOR SAMPLING CARBON DIOXIDE**

(71) Applicant: Meter-Q Solutions GmbH, 61169 Friedberg (DE)
(72) Inventor: Zajc, Achim, 35510 Butzbach (DE); Suhr, Jan, 35510 Butzbach (DE)
(74) Representative: Heeschen Pültz Patentanwälte PartGmbB

(57) **Abstract**

The invention relates to an extraction probe (100) for sampling carbon dioxide flowing in a pipeline (200), comprising: an inlet opening (110) configured to be arrangeable in the pipeline (200) and to sample carbon dioxide flowing in the pipeline (200); an extraction line (130) fluidly connected to the inlet opening (110), the extraction line (130) being configured to direct the sampled carbon dioxide to a measuring unit (300); at least one heating unit (140) configured to heat the sampled carbon dioxide flowing in the extraction line (130) to a predetermined temperature, wherein the predetermined temperature is higher than a temperature of carbon dioxide corresponding to a critical point of carbon dioxide.

## Description

The invention relates to an extraction probe and a method for sampling carbon dioxide flowing in a pipeline, a system for measuring carbon dioxide as well as a computer program product.

With the EU Green Deal and the German Climate Protection Act, the EU and Germany have committed to climate neutrality by 2050 and 2045 respectively. In order to achieve this ambitious goal, the industry is reliant on a wide range of climate protection technologies and corresponding political framework conditions.

The sectors with high and unavoidable long-term carbon dioxide or CO₂ emissions in the long term include cement, lime fertilizer and waste incineration. Even if every effort is made to reduce CO₂ emissions, a proportion will remain that cannot be avoided with the processes used today. To achieve the climate targets and not jeopardize industrial value creation there is no way around capturing of these unavoidable quantities of CO₂ with subsequent storage and utilization.

CO₂ capture and subsequent utilization or storage requires the transport of CO₂ from the source to the sink. Promising candidates for transport of CO₂ are pipelines, trains and ships. In the medium and long term, the majority of CO₂ transport in view of the expected quantities may be transported by pipeline. Each of the transport options has different technical requirements for the respective infrastructure and the CO₂ in terms of its pressure, temperature and purity. These determine the cost-effectiveness and efficiency of CO₂ transport.

For transport by rail or barge, the CO₂ is cooled to temperatures of -20 to -30 °C and transported at a comparatively moderate pressure of 15 to 20 bar. This operating point, at which the CO₂ is present as a liquid in equilibrium with a low gas volume, is a compromise in terms of the transport volume and the structure of the CO₂ tanks. Significantly lower CO₂ temperatures of -45 °C to -55 °C and a lower pressure are aimed for when transporting CO2 by ocean-going vessel.

In comparison, transport in CO₂ pipelines must be transported at ambient temperatures. As a rule, significantly higher pressures of over 100 bar (or up to a maximum of 200 bar for transport) must be set in order to transfer the CO₂ into the dense phase. This enables efficient transport even in very large quantities. Further details of reducing CO₂ are found in "Anforderungen an eine CO2-Infrastruktur in Deutschland", Verein Deutscher Zementwerke e.V. (VDZ), published 18 March 2024.

CO₂, e.g. from producers, directed to such pipelines as well as CO₂ already flowing in the pipelines need to meet predetermined requirements relating to the purity and composition of the gas often settled by the government. Accordingly, in order to control these requirements, measurements must be conducted when directing CO₂ into pipelines, exchanging CO₂ with other CO₂ pipelines or networks and the like. One option for controlling is to measure CO₂ in its gaseous phase with respective measuring units, wherein it is an essential condition that the CO₂ is in its gaseous form. Therefore, measurement devices are required which guarantee a gaseous form of the CO₂.

It is an objective of the present invention to provide an extraction probe and a method for sampling carbon dioxide and a system for measuring carbon dioxide which at least improve or even eliminate the aforementioned disadvantages. In particular, it is an objective of the present invention to provide a simplified and cost-efficient extraction probe, method and measuring system.

The objective is solved according to a first aspect by an extraction probe for sampling carbon dioxide flowing in a pipeline. The extraction probe comprises an inlet opening configured to be arrangeable in the pipeline and to sample carbon dioxide flowing in the pipeline. Furthermore, the extraction probe comprises an extraction line fluidly connected to the inlet opening, the extraction line being configured to direct the sampled carbon dioxide to a measuring unit. The measuring unit may be directly connected with the extraction line, e.g., by an interface or may have one or more units such as a later introduced pressure reduction unit and/or a further line. The extraction probe further comprises at least one heating unit configured to heat the sampled carbon dioxide flowing in the extraction line to a predetermined temperature, wherein the predetermined temperature is higher than a temperature of carbon dioxide corresponding to a critical point of carbon dioxide.

The inventors have found that heating the sampled carbon dioxide is already possible when it flows in the extraction line. By heating the sampled carbon dioxide above the critical point, the carbon dioxide is in the gaseous phase and thus ready for measurement by means of the measuring unit. This allows omitting an evaporator or the like in the measuring unit or between the extraction probe and the measuring unit. Therefore, the extraction probe can provide the carbon dioxide in the gaseous state to the measuring unit independently of the state of the carbon dioxide in the pipeline. Since parameters such as pressure and temperature of the carbon dioxide flowing in the pipeline may slightly vary, there is a risk that the carbon dioxide flowing in the pipeline is not suitable for being measured by the measuring unit. Due to the extraction probe heating the sampled carbon dioxide above the critical point, the sampled carbon dioxide is guaranteed to be in a state suitable for measurement. In addition, the absence of an evaporator reduces the overall costs and maintenance measures.

Sampling in the context of this invention may be regarded as extracting carbon dioxide from the pipeline.

The critical point of carbon dioxide may be at 31 C° and a pressure of 73.9 bar. Since the carbon dioxide may be transported by the pipeline at ambient temperature, if the pressure of the carbon dioxide in the pipelines is around 70 bar, the carbon dioxide may have the mixed state and may be liquid and gaseous. By increasing the temperature above the critical point, the carbon dioxide is guaranteed to be in its gaseous state for measurement purposes.

The measuring by means of the measuring unit may be performed at lower pressures compared to the pressure of the carbon dioxide flowing in the pipeline. Lowering the pressure of the gaseous carbon dioxide may be achieved by means of a pressure reduction unit. Said pressure reduction unit may be comprised by the extraction probe or the measuring unit.

The heating unit may be configured to heat the sampled carbon dioxide such that the carbon dioxide is in its gaseous state.

The heating unit may be configured to heat the sampled carbon dioxide to the predetermined temperature such that the heated carbon dioxide is received by the measuring unit at or with the predetermined temperature. In addition or as an alternative, the heating unit may be configured to maintain the temperature of the heated carbon dioxide at or above the predetermined temperature until the carbon dioxide is received by the measuring unit. The heating unit may be configured to heat the carbon dioxide such that the carbon dioxide is received by the pressure reduction unit in its gaseous phase. The pressure reduction unit may be arranged upstream of the measuring unit. The pressure reduction unit may be the first unit of the measuring unit to receive the sampled carbon dioxide.

The following definition of upstream and downstream may be applied. A fluid may flow along a flow direction, e.g., in a pipeline. The fluid may be output into the pipeline by a source and may be guided by the pipeline to a final destination. A unit being positioned in the pipeline and thus between the source and the final destination may be regarded as being positioned downstream of the source and upstream of the final destination.

The extraction probe may further comprise an outlet opening fluidly connected to the extraction line and configured to be arrangeable in the pipeline, the outlet opening being configured to release at least a portion of the sampled carbon dioxide into the pipeline. As an alternative, the outlet opening may be connected to another line of the extraction probe configured to release at least a portion of the sampled carbon dioxide into the pipeline. The extraction line may have at least one interface via which the carbon dioxide is at least partially directable to the measuring unit. The heating unit may be configured to heat the sampled carbon dioxide at least along a first section of the extraction line, the first section extending between the inlet opening and the interface. The extraction line may directly connect the inlet opening with the outlet opening. The first section may be a section of the extraction line extending from the inlet opening to the interface. As an alternative, the first section may extend to the interface or may incorporate the same and may have the opposite end thereof between the interface and the inlet opening along the extraction line. By heating only the first section of the extraction line, energy consumed for the heating may be reduced compared to the case of heating the whole extraction line.

The extraction line may be a single line or may be comprise two or more extraction lines fluidly connected with each other.

The heating unit may be configured to heat the sampled carbon dioxide to the predetermined temperature such that the heated carbon dioxide has at least the predetermined temperature at the interface.

The extraction probe may further comprise a plurality of heating units configured to heat the sampled carbon dioxide flowing in the extraction line to the predetermined temperature, wherein the heating units of the plurality of heating units are arranged along a flow direction of the sampled carbon dioxide flowing in the extraction line.

The heating units of the plurality of heating units may be at least partially arranged adjacent to each other and/or spaced apart from each other by a predetermined distance along the flow direction.

The extraction probe may have a thermal isolation to reduce any thermal exchange between the extraction line, an environment, the pipeline and/or the measuring unit. Also, the thermal isolation may reduce or eliminate heat dissipation to the environment from the heating unit.

Features described with reference to the heating unit may also apply to the plurality of heating units.

The heating unit may be comprised by the extraction line. The heating unit may be in contact with the carbon dioxide flowing in the extraction line.

The heating unit may be in direct contact with the extraction line and/or may be attached to the extraction line.

The heating unit may be arranged such that, when the extraction probe is arranged on and/or connected to the pipeline, the heating unit is spaced apart from the pipeline.

The pipeline may be configured to transport the carbon dioxide in a gaseous and/or liquid state.

The extraction probe may comprise a temperature sensor configured to determine a temperature of the sampled carbon dioxide, wherein the heating unit is configured to heat the sampled carbon dioxide based on the temperature determined by the temperature sensor. The measuring unit may comprise another temperature sensor to determine the temperature of the carbon dioxide in the measuring unit and/or received by the measuring unit.

The extraction probe may further comprise a pressure sensor to determine a pressure of the sampled carbon dioxide in the extraction line, wherein the heating unit is configured to heat the sampled carbon dioxide based on the pressure determined by the pressure sensor.

The extraction probe may be configured such that at least the inlet opening and the outlet opening are arrangeable in the pipeline, the extraction probe being configured such that the outlet opening is arrangeable downstream of the inlet opening along a flow direction of the carbon dioxide flowing in the pipeline. The extraction line may have the interface through which the carbon dioxide may at least partially be directable to the measuring unit for measuring the carbon dioxide. The extraction line may directly connect the inlet opening with the outlet opening.

By means of the extraction probe, a "loop" or circuit may be provided in which the carbon dioxide is extracted from the pipeline, particularly at a high pressure and/or flow velocity from the inlet opening, and the carbon dioxide may be directed to the outlet opening via the extraction line. By means of the interface, a portion of the extracted carbon dioxide may be extracted from the extraction line and directed to the measuring unit for measuring the carbon dioxide. Accordingly, already at the interface, only a reduced portion of the carbon dioxide may be extracted and directed to the measuring unit or the pressure reduction unit for reducing a pressure of the extracted carbon dioxide. The remaining portion of the carbon dioxide in the extraction line at high pressure may be directed back to the pipeline. Accordingly, the amount of carbon dioxide that is extracted for measurement is reduced. A further advantage is that the pressure and/or the flow velocity of the carbon dioxide in the pipeline are used. The arrangement of the inlet and outlet openings in the pipeline may cause a pressure differential between the inlet and outlet openings, in particular due to a stagnation pressure at the inlet opening and a dynamic pressure reduction at the outlet opening. This pressure differential may cause the carbon dioxide to be extracted through the inlet opening, flow through the extraction line, and to the outlet opening, as the pressure difference may cause a flow or flow velocity within the extraction probe. For example, the pressure of the carbon dioxide within the pipeline may be 100 bar, so that there may be a stagnation pressure of 5 bar of the carbon dioxide at the inlet opening, thus a total pressure of 105 bar at the inlet opening, and a dynamic pressure of 2 bar of the carbon dioxide at the outlet opening, thus a total pressure of 98 bar at the outlet opening. Thus, there is a pressure difference of 7 bar between the inlet and outlet openings.

The extraction probe may be configured such that the pressure differential between a first pressure of the carbon dioxide extracted by means of the extraction probe at the inlet opening and a second pressure of the carbon dioxide directed by means of the outlet opening may be generated, wherein the pressure differential may be generated such that the carbon dioxide extracted by means of the extraction probe flows through the extraction probe at a predetermined flow velocity. The predetermined flow velocity may be determined based on the flow path and/or the measuring objective of the measuring unit.

The inlet opening may be configured by an extraction cross-section for extracting the carbon dioxide from the pipeline and/or the outlet opening may be configured by an output cross-section for at least partially outputting the extracted carbon dioxide into the pipeline. The extraction probe may configured such that the extraction cross-section and/or the output cross-section are arrangeable perpendicular to a flow direction in the pipeline.

The extraction probe may be configured such that the extraction cross-section faces the carbon dioxide flowing along the flow direction in the pipeline and/or the output cross-section faces away from the carbon dioxide flowing along the flow direction in the pipeline.

The extraction probe may further comprise a line configured to direct the extracted carbon dioxide from the interface to the pressure reduction unit, the pressure reduction unit being configured to reduce a pressure of the carbon dioxide to a predetermined pressure. The extraction probe and/or the measuring unit may further comprise a line by means of which the carbon dioxide at the reduced pressure is directed to the measuring unit.

The heating unit may be configured to heat one or more units of the extraction probe, in particular to achieve and/or maintain the predetermined temperature of the sampled carbon dioxide.

The measuring unit may additionally comprise a heating unit to at least maintain the predetermined temperature of the carbon dioxide in the measuring unit.

The heating unit may be configured to heat the sampled carbon dioxide at least to the temperature corresponding to the critical point. Therefore, the heating unit may be configured to heat the sampled carbon dioxide such that a temperature of the carbon dioxide is higher than the temperature corresponding to the critical point. Accordingly, the temperature of the heated carbon dioxide may vary in the extraction line and/or the measuring unit, however, may be above the temperature corresponding to the critical point.

The pressure reduction unit may be configured to heat the sampled carbon dioxide, in particular of the sampled carbon dioxide with reduced pressure.

The objective is solved according to a second aspect by a method for sampling carbon dioxide flowing in a pipeline, comprising the steps of:
- sampling carbon dioxide flowing in the pipeline with an extraction probe according to the first aspect;
- heating the sampled carbon dioxide flowing in the extraction line to a predetermined temperature, wherein the predetermined temperature is higher than a temperature of carbon dioxide corresponding to a critical point of carbon dioxide;
- directing the heated carbon dioxide to a measuring unit for measuring the sampled carbon dioxide.

The method may further comprise the step of measuring the sampled carbon dioxide, in particular via the measuring unit.

The method may at least be partially computer implemented.

The steps of the method may be performed by the corresponding units of the extraction probe according to the first aspect. Device features of the extraction probe may be executed as method steps of the method of the second aspect and are thus not repeated here.

The objective is solved according to a third aspect by a system for measuring carbon dioxide flowing in a pipeline, comprising an extraction probe according to the first aspect and a measuring unit configured to measure the sampled carbon dioxide. The measuring unit and the extraction probe may be fluidly connected.

The measuring unit and/or the extraction probe may further comprise a processor and/or a storage unit. The storage unit may be configured to store a computer program product as defined below. The processor(s) may be configured to execute the computer program of the computer program product.

The measuring unit may be configured to measure any property of the carbon dioxide. The measuring unit may be configured to execute the measurement at different pressures of the carbon dioxide and even at a pressure similar or identical to the pressure of the carbon dioxide in the pipeline. In order to reduce the pressure, the measuring unit may comprise a pressure reduction unit to reduce a pressure of the carbon dioxide.

The objective is solved according to a fourth aspect by a computer program product, comprising instructions to cause the extraction probe of the first aspect and/or system of the third aspect to execute the steps of the method according to the second aspect.

Preferred embodiments are exemplified with reference to the accompanying figures. It shows:
- Fig. 1: a schematic illustration of an extraction probe extracting carbon dioxide of a pipeline and connected to a measuring unit;
- Fig. 2: a detailed schematic illustration of the extraction probe; and
- Fig. 3: a schematic illustration of a method for sampling carbon dioxide.

In the figures, identical or substantially functionally identical or similar elements are designated with the same reference signs.

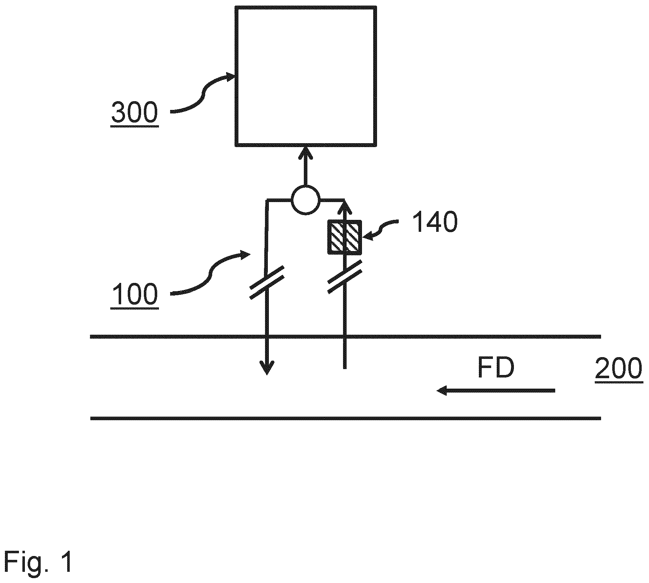

Fig. 1 shows an extraction probe 100 arranged on and/or connected to a pipeline 200. The pipeline 200 transports carbon dioxide in a liquid state, a mixed state of the carbon dioxide being partly liquid and partly gaseous or gaseous state. The extraction probe 100 is partly inserted into the pipeline 200 to extract or sample the carbon dioxide flowing along a flow direction FD in the pipeline 200.

The extraction probe 100 is fluidly connected to a measuring unit 300 for measuring the sampled carbon dioxide.

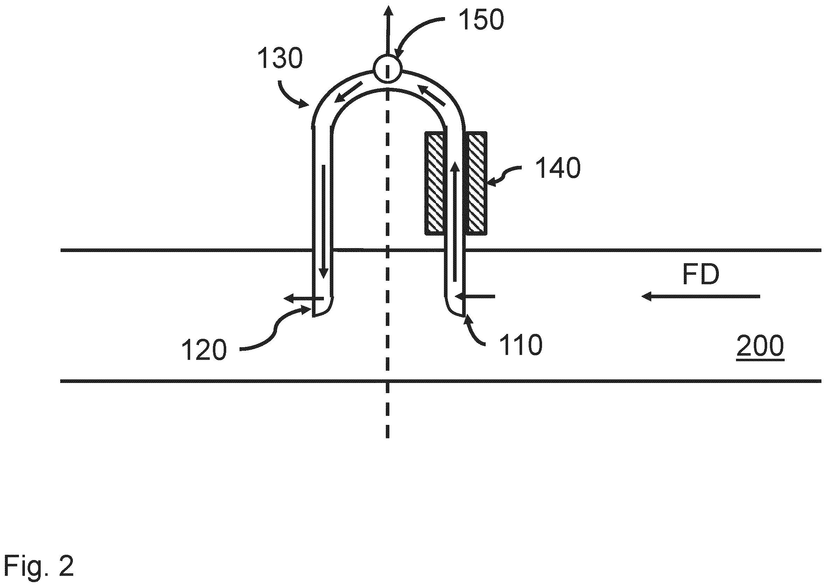

As illustrated in Fig. 2 in more detail, the extraction probe 100 comprises an inlet opening 110 arranged in the pipeline 200 and configured to sample carbon dioxide flowing in the pipeline 200. The arrows in Figs. 1 and 2 indicate a flow or stream direction FD. The extraction probe 100 further comprises an outlet opening 120. The inlet opening 110 and the outlet opening 120 are fluidly connected by an extraction line 130, wherein the inlet and outlet opening 110, 120 define respective end sections of the extraction line 130.

The extraction probe 100 may comprise a housing in which the extraction line 130 is at least partially arranged. The housing may be configured to be arranged on, coupled to and/or connected to the pipeline 200. The extraction probe 100 and/or the housing may have attachment means for attaching the extraction probe 100 to the pipeline 200. End sections of the extraction line 130 comprising the inlet and outlet openings 110, 120 may be arranged outside of the housing and/or extend through the housing such that said end sections may be inserted into the pipeline 200.

Furthermore, an interface 150 of the extraction probe 100 is illustrated. The interface 150 is configured to fluidly connect the extraction line 130 to the measuring unit 300, here by means of another line. A pressure reduction unit (not shown) may be arranged between the interface 150 and the measuring unit 300, wherein the pressure reduction unit is configured to reduce a pressure of the sampled carbon dioxide being directed to the measuring unit 300.

Since it is crucial for the measuring unit 300 that the carbon dioxide is in its gaseous state, the extraction probe 100 further comprises at least one heating unit 140 configured to heat the sampled carbon dioxide flowing in the extraction line 130 to a predetermined temperature, wherein the predetermined temperature is higher than a temperature of carbon dioxide corresponding to a critical point of carbon dioxide. By heating the carbon dioxide already in the extraction line to the predetermined temperature, the sampled carbon dioxide is guaranteed to be in its gaseous state. An evaporator between the extraction probe 100 and the measuring 300 can thus be omitted.

The heating unit 140 is exemplarily shown as heating a section of the extraction line 130 between the interface 150 and the inlet opening 110. Furthermore, the heating unit 140 is located outside of the pipeline 200 and spaced apart from it. The heating unit 140 is not limited thereto and may be in contact with the pipeline 200. Also, the heating unit 140 may extend along the extraction line to the interface 150 such that a cooling off of the sampled carbon dioxide is prevented. The heating unit 140 may be arranged in the housing. As an alternative, the heating unit 140 as illustrated in Fig. 2 may heat the sampled carbon dioxide higher than the critical point such that a cooling off of the sampled carbon until arriving at the interface would still result in the carbon dioxide being gaseous. The heating unit may be configured to heat not only the extraction line, but any further lines of the extraction probe 100 such that it may be guaranteed that the carbon dioxide is at the predetermined temperature. The pressure reduction unit may also be arranged inside of the housing. In general, the housing may be configured such that one or more of the units of the extraction probe 100 may be arranged at least partially inside of the housing. The heating unit 140 may be configured as an explosion protection and/or explosion proven.

Moreover, as shown in Figs. 1 and 2, since the inlet and outlet openings 110, 120 are directly connected by the extraction line 130, a "loop" of the sampled carbon dioxide is achieved. Since the carbon dioxide flowing in the pipeline 200 has a predetermined flow velocity, said flow velocity may be beneficially used to transport the sampled carbon dioxide along the extraction line 130 to the interface 150, wherein only a portion of the sampled carbon dioxide is directed to the measuring unit 300. The rest of the sampled carbon dioxide can flow to the outlet opening 120 and can be released into the pipeline 200. This also reduces the amount of carbon dioxide directed to the measuring unit 300 and consequently the amount to be released by the measuring unit 300 itself.

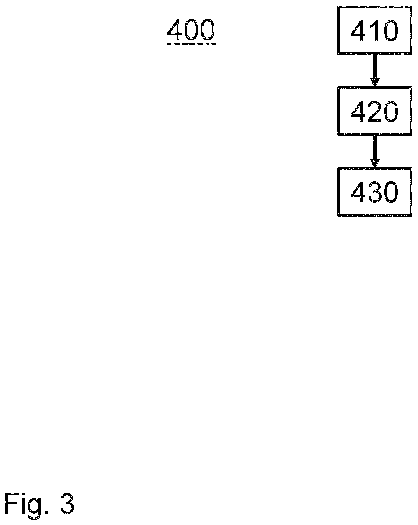

Fig. 3 shows a schematic method 400 for sampling carbon dioxide flowing in a pipeline 200. The method 400 comprises sampling 410 carbon dioxide flowing in the pipeline 200 with the extraction probe 100 and heating 420 the sampled carbon dioxide in the extraction line 130 to a predetermined temperature, wherein the predetermined temperature is higher than a temperature of carbon dioxide corresponding to a critical point of carbon dioxide. The method 400 further comprises directing 430 the heated carbon dioxide to a measuring unit 300 for measuring the sampled carbon dioxide. The method 400 may be stored as a computer program product on a storage. The method 400 may be executed by a system comprising the measuring unit 300 and the extraction probe 100 as, e.g., illustrated in Fig. 1.

### REFERENCE SIGNS

- 100: Extraction probe
- 110: inlet opening
- 120: outlet opening
- 130: extraction line
- 140: heating unit
- 150: interface
- 200: pipeline
- 300: measuring unit
- 400: method for sampling carbon dioxide
- 410: sampling carbon dioxide
- 420: heating the sampled carbon dioxide
- 430: directing the heated carbon dioxide to a measuring unit
- FD: flow direction

## Claims

1. Extraction probe (100) for sampling carbon dioxide flowing in a pipeline (200), comprising:
an inlet opening (110) configured to be arrangeable in the pipeline (200) and to sample carbon dioxide flowing in the pipeline (200);
an extraction line (130) fluidly connected to the inlet opening (110), the extraction line (130) being configured to direct the sampled carbon dioxide to a measuring unit (300);
at least one heating unit (140) configured to heat the sampled carbon dioxide flowing in the extraction line (130) to a predetermined temperature, wherein the predetermined temperature is higher than a temperature of carbon dioxide corresponding to a critical point of carbon dioxide.

2. Extraction probe (100) according to claim 1,
wherein the heating unit (140) is configured to heat the sampled carbon dioxide to the predetermined temperature such that the heated carbon dioxide is received by the measuring unit (300) at the predetermined temperature.

3. Extraction probe (100) according to claim 1 or 2, further comprising:
an outlet opening (120) fluidly connected to the extraction line (130) and configured to be arrangeable in the pipeline (200), the outlet opening (120) being configured to release at least a portion of the sampled carbon dioxide into the pipeline (200),
wherein the extraction line (130) has at least one interface (150) via which the carbon dioxide is at least partially directable to the measuring unit (300),
wherein the heating unit (140) is configured to heat the sampled carbon dioxide at least along a first section of the extraction line (130), the first section extending between the inlet opening (110) and the interface (150).

4. Extraction probe (100) according to claim 3,
wherein the heating unit (140) is configured to heat the sampled carbon dioxide to the predetermined temperature such that the heated carbon dioxide has the predetermined temperature at the interface.

5. Extraction probe (100) according to any of the previous claims, further comprising:
a plurality of heating units (140) configured to heat the sampled carbon dioxide flowing in the extraction line (130) to the predetermined temperature,
wherein the heating units of the plurality of heating units (140) are arranged along a flow direction of the sampled carbon dioxide flowing in the extraction line (130).

6. Extraction probe (100) according to claim 5,
wherein the heating units of the plurality of heating units (140) are at least partially arranged adjacent to each other and/or at least spaced apart from each other by a predetermined distance along the flow direction of the sampled carbon dioxide.

7. Extraction probe (100) according to any of the previous claims, further comprising:
a temperature sensor configured to determine a temperature of the sampled carbon dioxide,
wherein the heating unit (140) is configured to heat the sampled carbon dioxide based on the temperature determined by the temperature sensor.

8. Method (400) for sampling carbon dioxide flowing in a pipeline (200), comprising the steps of:
sampling (410) carbon dioxide flowing in the pipeline (200) by means of an extraction probe (100) according to any of claims 1 to 7;
heating (420) the sampled carbon dioxide in the extraction line (130) to a predetermined temperature, wherein the predetermined temperature is higher than a temperature of carbon dioxide corresponding to a critical point of carbon dioxide;
directing (430) the heated carbon dioxide to a measuring unit (300) for measuring the sampled carbon dioxide.

9. System for measuring carbon dioxide flowing in a pipeline (200), comprising:
an extraction probe (100) according to any of claims 1 to 7;
a measuring unit (300) configured to measure the sampled carbon dioxide.

10. Computer program product, comprising instructions to cause the extraction probe (100) according to any of claims 1 to 7 and/or the system of claim 9 to execute the steps of the method (400) of claim 8.
